(19) Europäisches Patentamt
European Patent Office
Office européen des brevets

(11) Publication number: 0 514 885 A2

(12) # EUROPEAN PATENT APPLICATION

(21) Application number: 92108566.8

(22) Date of filing: 21.05.92

(51) Int. Cl.$^5$: C07D 501/18, C07F 7/10

(30) Priority: 24.05.91 AT 1063/91

(43) Date of publication of application:
25.11.92 Bulletin 92/48

(84) Designated Contracting States:
AT BE CH DE DK ES FR GB GR IT LI LU NL PT SE

(71) Applicant: BIOCHEMIE Gesellschaft m.b.H.
A-6250 Kundl Tirol(AT)

(72) Inventor: Ascher, Gerd
Daxerfeld 3
A-6250 Kundl(AT)
Inventor: Egerbacher, Josef
Schönau 113
A-6323 Bad Häring(AT)
Inventor: Krenmüller, Franz, Ing.
Walther-Simmerweg 5
A-6330 Kufstein(AT)

(74) Representative: Kleine Deters, Johannes et al
c/o Sandoz Technology LTD., Patent- und Markenabteilung
CH-4002 Basel(CH)

(54) **New process for the production of 7-amino-3-azidomethyl-3-cephem-4-carboxylic acid.**

(57) The invention relates to a new process for the production of 7-amino-3-azidomethyl-3-cephem-4-carboxylic acid.

EP 0 514 885 A2

The invention relates to a new process for the production of 7-amino-3-azidomethyl-3-cephem-4-carboxylic acid of formula I

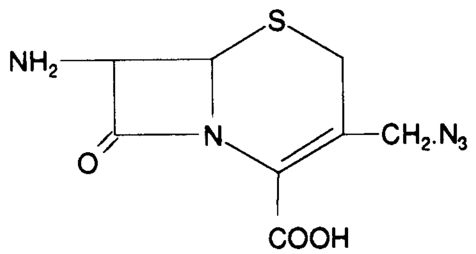

I

7-Amino-3-azidomethyl-3-cephem-4-carboxylic acid is an important starting material for pharmaceutically important cephalosporins bearing the azidomethyl group in 3-position.

The preparation of 7-amino-3-azidomethyl-3-cephem-4-carboxylic acid, abbreviated in the following to "azido-ACA", is described in literature, for example in EPA 127 992, DOS 2 101 221 and DOS 2 332 045. Due to the poor solubility of the salt-like azide employed, all these processes for the production of azido-ACA are generally performed only in an aqueous medium and at a high temperature. As is known, in cephalosporin chemistry, this leads to a large formation of by-products and low yield.

The low yield due to decomposition and the generally required excess of the salt-like azide lead to a high azide-concentration in the reaction solution, which lead to safety and ecological problems.

In accordance with the invention, the compound of formula I, is obtained in a high yield by reacting compounds of formula II

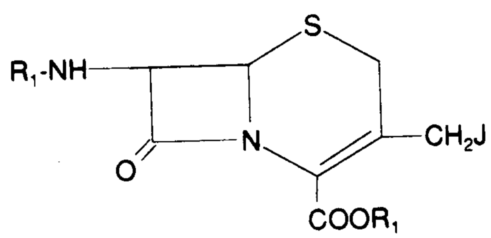

II

wherein $R_1$ is a silyl protecting group with a trialkyl$_{(1-4)}$silyl azide, preferably trimethylsilyl azide.

Examples of silyl protecting groups include trimethylsilyl, triethylsilyl, tri-n-propyl-silyl, tri-n-butylsilyl, methyldiethylsilyl, dimethylethylsilyl, phenyldimethylsilyl, tert. butyldiphenylsilyl, tert. butyldimethylsilyl and triphenylsilyl. The trimethylsilyl group is preferred.

The compounds of formula II are known and may be produced by the process disclosed in AT-Patent 382 875. For example, aminocephalosporanic acid is suspended in an inert solvent and heated at reflux with an excess of silylation agent. Suitable solvents for silylation are in particular halogenated hydrocarbons. With high-boiling solvents, disilylation is effected by excessive heating in the solvent, and with low-boiling solvents silylation may be accelerated by adding organic acids, such as trifluoroacetic acid or trichloroacetic acid. Silylation can also be effected by using the nitrogen-containing silylation catalysts described in EPA 043 630. The silylation agents that may be used are preferably 1,1,1,3,3,3-hexamethyldisilazane alone or 1,1,1,3,3,3-hexamethyldisilazane in admixture with other silylation agents, such as trimethylchlorosilane, N,0-bis-(trimethylsilyl)acetamide, N-(trimethylsilyl)acetamide or the trifluoro analogues thereof or also bistrimethylsilyl urea. The disilylated compounds thus obtained are subsequently reacted with a trialkylsilyl iodide, preferably trimethyliodosilane, to form compounds of formula II as described in AT-Patent 382 875.

The process according to the invention may be carried out by a process wherein a compound of formula II is reacted preferably in situ, i.e. without isolating it from the reaction mixture, with a trialkylsilyl azide, preferably with azidotrimethylsilane. The reaction may be carried out in organic solvents, leading to better execution of the reaction, higher stability of the substances taking part therein, and thus higher yields, as well as higher quality of the product. The reaction may be carried out for example in the same solvent that was used for the previous stages, or after adding an additional solvent. Examples of suitable solvents are halogenated hydrocarbons, e.g. methylene chloride or acetonitrile. The reaction temperature is not critical due to the use of organic solvents instead of the aqueous systems employed in previous processes. The reaction may therefore also be carried out at room temperature or at a low temperature; the preferred

temperature range is 0-20 ° C.

The reaction mixture may be worked up under acidic or alkaline conditions. A product of especially high purity may be obtained by using solvents which are immiscible with water for the reaction, with subsequent aqueous/alkaline working up. For this, strong organic bases are preferably used, for example guanidines and amidines, such as DBU (1,8-diazabicyclo[5.4.0]undec-7-ene) and DBN (1,5-diazabicyclo[4.3.0]non-5-ene).

A further important advantage of this invention is the higher purity of the isolated product, compared with those obtained from the aqueous processes.

As mentioned the compound of formula I is an important known starting material for the production of valuable cephalosporin antibiotics e.g. by acylating e.g. with an appropriate acylating agent e.g. as disclosed in the patent filings referred to above.

In the following example, which illustrates the invention more fully but in no way limits its scope, all temperatures are given in degrees celsius.

**Example: 7-amino-3-azidomethyl-3-cephem-4-carboxylic acid**

A solution of 0.183 moles of 7-trimethylsilylamino-3-iodomethyl-3-cephem-4-carboxylic acid trimethylsilyl ester in 1000 ml of methylene chloride is mixed with 31.7 g (0.275 moles) of azidotrimethylsilane and stirred for 1 hour at 10 ° . The reaction solution is subsequently discharged into water/DBU. After separating the phases, the pH is adjusted to 3.0 with hydrochloric acid and the suspension is stirred for 1 hour at 0 ° . The deposit is filtered off, washed with water and dried.

The title compound obtained in this way has the following NMR and IR data:

$^1$H-NMR (90 MHz, $D_2O/K_2CO_3$): 5.08 (d, 1H, J = 6Hz, H-7); 4.75 (d, 1H, J = 6Hz, H-6); 4.10 (s, 2H, $-CH_2-N_3$); 3.70 (d, 1H, J = 18Hz, H-2); 3.36 (d, 1H; J = 18Hz, H-2').

IR (KBr)/($cm^{-1}$): 2110 (s, azide); 1795 (s, $\beta$-lactam-CO).

## Claims

1. A process for the production of the compound of formula I

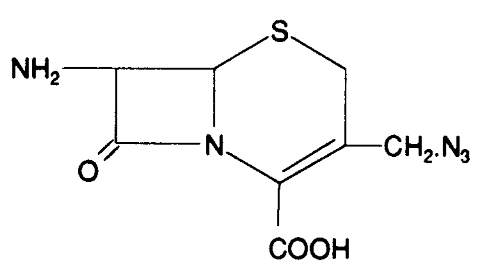

I

which comprises reacting a compound of Formula II

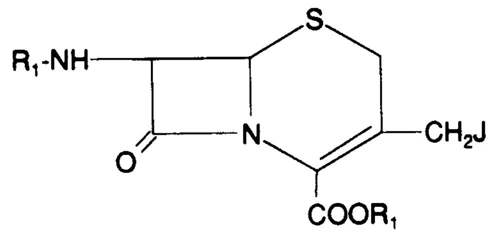

II

wherein $R_1$ is a silyl protecting group with a trialkyl($C_{1-4}$)silyl azide.

2. A process according to claim 1 wherein trimethylsilylazide is used as a trialkyl($C_{1-4}$)silyl azide.

3. A process for the production of a 7-acylamino-3-cephem-4-carboxylic acid which comprises acylating the compound of formula I produced by a process of claim 1 or 2.